# EUROPEAN PATENT APPLICATION

(11) **EP 1 013 767 A1**
(43) Date of publication of application: **28.06.2000**
(21) Application number: 98124525.1
(22) Date of filing: 22.12.1998
(51) Int. Cl.: C12N 15/53, C12Q 1/68

(54) **Method of screening for agrochemicals**

(71) Applicant: AMERICAN CYANAMID COMPANY, Madison, New Jersey 07940 (US); Institut für Pflanzenbiochemie, 06120 Halle/Saale (DE)
(72) Inventor: Wasternack, Claus, Dr., 06122 Halle / Saale (DE); Hause, Bettina, Dr., 06122 Halle / Saale (DE); Besser, Katrin, 35390 Giessen (DE)
(74) Representative: Wibbelmann, Jobst, Dr., Dipl.-Chem.

(57) **Abstract**

The invention relates to a method of testing agrochemicals for their capability to induce SAR in plants said method comprising:
(a) contacting a plant or plant part comprising a gene fragment coding for a lipoxygenase (LOX) the expression of which being inducible by salicylate, but not by naturally occurring plant pathogens, with an agrochemical; and
(b) assaying the expression of said gene fragment, wherein the expression of said gene fragment indicates that the agrochemical has the capability to induce SAR. This method allows i.a. to screen for SAR inducing compounds in different plant species. Furthermore, kits for use in said methods are provided as well as a process for providing compounds having the capability of inducing SAR in plants, comprising the steps of
   (a) generating a synthetic combinatorial library of different chemical compounds, and
   (b) screening the compounds of said library with the screening method according to this invention.

## Description

### BACKGROUND OF THE INVENTION

This invention concerns a method of testing an agrochemical for its capability to induce SAR in plants and to the use of said method for screening for agrochemicals having the capability to induce SAR in plants.

In the last decade convincing data have been accumulated that salicylic acid (SA) is essential for the establishment of systemic acquired resistance (SAR) of dicotyledonous plants against a broad range of pathogens (e.g. Yang et al., Genes and Development 11, 1621-1639 (1997)). In contrast, other plant defence reactions such as the wound response including expression of the proteinase inhibitor 1 and 2 gene, are inhibited by SA or its acetyl derivative aspirin (e.g. Doares et al., Plant Physiol. 108, 1741-1746 (1995)).

Lipoxygenases (LOX) are assumed to be involved in different environmentally and developmentally regulated processes including the establishment of plant resistance (Rosahl, Z. Naturforsch 51 C, 123-138 (1996)). In plants different LOX isoenzymes catalyse either the formation of 9-(S)-hydroperoxy-10-(E),12-(Z),15-(Z)-octadecatrienoic acid (9-HOTE) or 13-(S)-hydroperoxy-9-(Z),11-(E),15-(Z)-octadecatrienoic acid (13-HOTE) which is the precursor of jasmonic acid.

In barley at least three LOX isoenzymes are strongly induced by jasmonates (Feussner et al., Plant J. 7, 949-957 (1995)). A cDNA coding for the most abundantly accumulating jasmonate-responsive LOX of a molecular mass of 106 kDa was recently isolated and designated as *LOX2:Hv:1* (Vörös et al. , Eur. J. Biochem. 251, 36-44 (1998)). It was identified as a LOX2-type enzyme.

The US Patent 5,614,395 discloses a method for screening for SAR inducing agrochemicals with the aid of genetically modified plants containing a chimeric gene which responds on SAR inducing compounds, which is disclosed as a wheat gene such as *WCI-1*.

However, this method is limited to those plant species which are susceptible for the chimeric gene, in particular to wheat.

I. Feussner et al., Botanica Acta 110 (2) 1997, pp. 101-108 disclose that a certain 95 kDa LOX gene is responsive on a SAR inducing compound such as 2,6-dichloroisonicotinic acid. However, the expression of this gene fragment is also induced upon inoculation with mildew. Therefore, this gene fragment cannot be used to screen for SAR inducing compounds in a substantially natural environment like in the glass house or on the field.

The International Patent application WO 98/00023 suggests a method of screening compounds for resistance-inducing activity by the detection of the expression of a plant defensin gene such as *PDF 1*.

However, the genes disclosed there are induced by plant pathogens such as *Alternaria brassicicola* or *Botrytis cinera* but not by SAR inducing agrochemicals such as salicylic acid or dichloroisonicotinic acid.

The generation of synthetic combinatorial libraries is well known for example from EP 0 734 530, EP 0 816 309 and EP 0 818 431. However, up to now it was only possible to screen such libraries in order to select compounds with pharmaceutical properties. Therefore, it was highly desirable to develop a high through-put screening method which allows to generate lead compounds for the agricultural research, in particular for SAR inducing compounds.

### SUMMARY OF THE INVENTION

The present invention is based on the most surprising finding that there exist genes encoding lipoxygenase enzymes the expression of which is inducible by salicylate, but not by naturally occurring plant pathogens. This finding enables the establishment of novel methods for testing an agrochemical for its capability to induce SAR in plants and for screening a multiplicity of agrochemicals for compounds or compositions having the capability to induce SAR in plants.

The present invention relates to a method of testing an agrochemical for its capability to induce SAR in plants said method comprising:
(a) contacting a plant or plant part comprising a gene fragment coding for a lipoxygenase (LOX) the expression of which being inducible by salicylate, but not by naturally occurring plant pathogens, with the agrochemical; and
(b) assaying the expression of said gene fragment, wherein the expression of said gene fragment indicates that the agrochemical has the capability to induce SAR.

Furthermore, this method according to the present invention allows to screen SAR inducing compounds in different plant species as for example wheat, barley and rice; to determine the presence of SAR-inducing compounds in a sample; to determine the concentration of a SAR-inducing compound in a sample; to compare two SAR-inducing compounds for their relative capabilities to induce SAR in plants; and to test compounds and compositions for their capability to change the SAR inducing effect of an agrochemical.

A further aspect of the present invention relates to a kit for use in the methods of the present invention.

Another aspect of the present invention relates to a process for providing compounds having the capability of inducing SAR in plants which comprises the steps of
(a) generating a synthetic combinatorial library of different chemical compounds, and
(b) screening the compounds of said library with the screening method according to this invention.

A further aspect of the invention are the novel compounds which are obtainable with the aid of the method according to this invention.

These and other objects and features of the invention will become more apparent from the detailed description set forth hereinbelow.

### BRIEF DESCRIPTION OF THE DRAWINGS

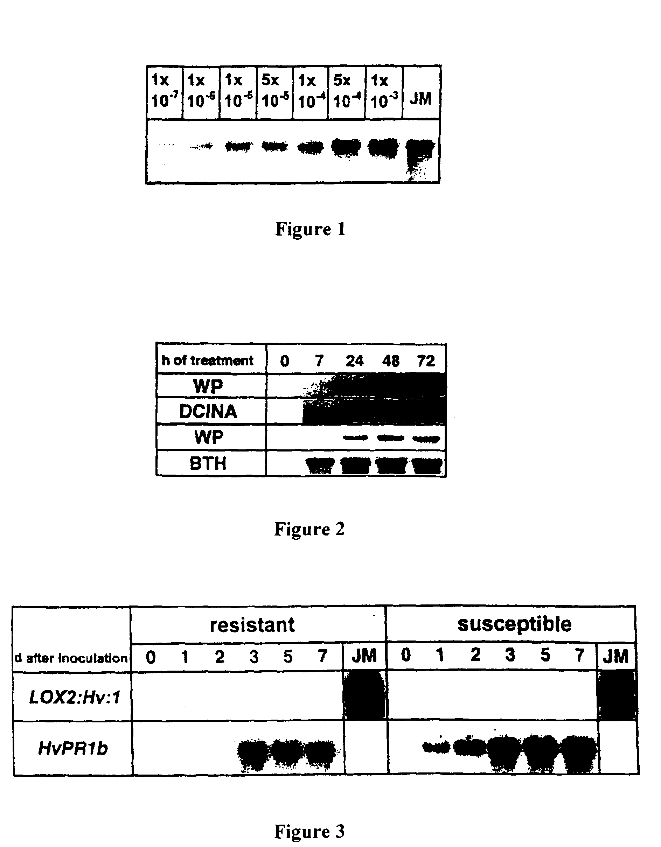

FIG. 1: Northern blot analysis of *LOX2:Hv:1* mRNA accumulation in primary leaves of barley cultivar Salome treated with sodium salicylate at the indicated concentrations for 24 hours. 10 µg total RNA was loaded per lane. JM = leaf segment floated on 45 µM of jasmonic acid methyl ester as reference control.
FIG. 2: Northern blot analysis of *LOX2:Hv:1* mRNA accumulation in primary leaves of barley upon treatment with plant agrochemicals. 10 µg total RNA was loaded per lane. Treatment of primary leaves of the cultivar Machuria (susceptible line) with DCINA = 2,6-dichloroisonicotinic acid, formulated as 25 % active ingredient with a wettable powder (WP) or BTH = benzo-[1,2,3]-thiadiazole-7-carbothioic acid S-methyl ester applied at a concentration of 1 mM by spraying a formulation with WP in distilled water; WP = corresponding control treatment with wettable powder alone.
FIG. 3: Northern blot analysis of *LOX2:Hv:1*. and *PR1b* mRNA accumulation in primary leaves of barley cultivar Salome (resistant) and cultivar Pallas (susceptible) after inoculation with *Blumeria graminins f*. *sp. hordei*. 10 µg total RNA was loaded per lane. JM = *LOX2:Hv:1* expression in leaf segment floated on 45 µM of jasmonic acid methyl ester for 24 hours as reference control.

### DETAILED DESCRIPTION OF THE PREFERRED EMBODIMENTS

It has now been found that the capability of agrochemicals to induce SAR in plants can be efficiently tested by
(a) contacting a plant or plant part comprising a gene fragment coding for a lipoxygenase (LOX) the expression of which being inducible by salicylate, but not by naturally occurring plant pathogens, with an agrochemical; and
(b) assaying the expression of said gene fragment.

It is an essential feature of the present invention that the salicylate-inducible expression of the said gene fragment in the said plant is not inducible by naturally occurring plant pathogens. This feature appears to be particularly valuable if it is intended to perform the methods of the present invention in an environment where plant pathogens may be present. This will particularly be the case when using plants in a green house or on the field for performing the methods of the present invention. In these cases, said specific feature will ensure that any expression of the said gene fragment observed will only be attributable to the presence of a compound or composition having the capability to induce SAR in plants.

By use of the method of the present invention, a multiplicity of chemical compounds and compositions may be screened to identify compounds and/or compositions having the capability to induce SAR in plants. In a particular embodiment said multiplicity of chemical compounds or compositions may be provided, for example, in form of a combinatorial library such as exemplified in EP 0 734 530, EP 0 816 309 and EP 0 818 431.

Alternatively, the said method may also be used for determining the presence of compounds capable of inducing SAR in plants in a sample, wherein in the above step (a) the plant or plant part is contacted with an aliquot of the sample in place of the agrochemical. The expression of the said gene fragment then indicates the presence of one or more compounds capable of inducing SAR in plants in the sample.

In a preferred embodiment of the methods of the present invention the plant used is a plant wherein the salicylate-inducible expression of the said gene fragment is tissue-specific, and, in particular, specifically occurs in the plant leaves.

In the present context, the phrase "naturally occurring plant pathogens" includes all kinds of phytopathogenic microorganisms, in particular phytopathogenic fungi, bacteria and viruses.

The phrase "plants" as used hereinabove and hereinbelow includes all kinds of plants and all plant parts, in particular foliage, roots and seeds. Preferably the said plant or plant part is selected from the group consisting of a plant protoplast, a plant cell, plant tissue, a developing plantlet, a plant leaf, an immature whole plant and a mature whole plant. In particular, the said plant or plant part, in particular plant cell or plant leaf, is or is derived from a monocotyledonous plant, such as cereals, particularly rice and preferably barley, as well as dicotyledonous plants.

The salicylate-inducible gene fragment of step (a) may be any gene fragment encoding a lipoxygenase enzyme the expression of which being inducible by salicylate, but not by naturally occurring plant pathogens. This definition comprises any naturally occurring gene fragments exhibiting these characteristics as well as not-naturally occurring gene fragments, the sequence of which has substantial homology with the sequence of any of said naturally occuring gene fragments, variants thereof as well as gene fragments comprising one or more portions of the aforementioned gene fragments and variants wherein all these gene fragments and variants maintain the characteristic indicated above, i.e. an expression which is inducible by salicylate, but not by naturally occurring plant pathogens.

In a preferred embodiment of the present invention, the salicylate-inducible gene fragment of step (a) corresponds to a gene fragment consisting of, essentially consisting of or comprising the nucleotide sequence of a cDNA derivable from mRNA transcribed from *LOX2:Hv:1* as disclosed by Vörös et al., Eur. J. Biochem. 251, 36-44 (1998), which is hereby incorporated by reference. Said cDNA comprises 3073 nucleotide base pairs coding for a protein of 936 amino acid residues with a molecular mass of 106 kDa.

In an alternative embodiment, the said gene fragment is a gene fragment, the sequence of which exhibits substantial homology with the sequence of *LOX2:Hv:1*, or a variant thereof or comprises one or more portions of the sequence of *LOX2:Hv:1*, of a sequence having substantial homology thereto or a variant thereof. Due to substantial homology, the expression of many of said gene fragments or variants may be detected by use of nucleotide probes comprising the cDNA sequence of *LOX2:Hv:1* or portions thereof. This is particularly true for a great number of homologous sequences and variants which may be found in plant species other than barley, and in particular in other cereals.

Accordingly, the terms also include sequences that can substantially hybridize to the gene sequence of *LOX2:Hv:1*, in particular under high-stringency conditions. They also include DNA which hybridizes to the DNA of *LOX2:Hv:1*. or a part of it and which codes for at least part of the gene sequence.

The phrase "variant thereof" as used hereinabove and hereinbelow means any substitution, variation, modification, insertion, deletion or addition of one or more nucleotides from or to the nucleotide sequence of a naturally occurring salicylate-inducible gene fragment coding for a lipoxygenase enzyme, the expression of which not being inducible by naturally occurring plant pathogens, for example *LOX2:Hv:1*, provided that the expression of the variant being still inducible by application of SAR inducing compounds, but not by naturally occurring plant pathogens. The phrase also includes variants comprising or essentially consisting of nucleotide sequences that can substantially hybridize to the nucleotide sequence of the naturally occurring parent gene fragment. It also includes DNA which hybridizes to the nucleotide sequence of the parent gene fragment, and which codes for at least part of the lipoxygenase gene product of the said gene fragment. Preferably, such hybridisation occurs at, or between low and high stringency conditions, and in particular at high stringency conditions. As a rule low stringency conditions can be defined as 3 x SSC at ambient temperature to 65°C and high stringency conditions as 0.1 x SSC at 65°C. SSC is the abbreviation of a 0.15M sodium chloride, 0.015M trisodium citrate buffer.

The phrase "substantial homology" as used hereinabove and hereinbelow embraces homology with respect to at least the essential nucleotide/s of the parent gene sequence encoding a lipoxygenase, e.g., *LOX2:Hv:1*, provided that the homologous sequence acts as the parent lipoxygenase gene fragment and, in particular, the expression thereof is still inducible by SAR inducing compounds, but not by naturally occurring plant pathogens. Typically, homology is shown when 60 % or more of the nucleotides are common with the naturally occurring parent gene fragment, more typically 65 %, preferably 70 %, more preferably 75 %, even more preferably 80 or 85 % and in particularly preferred are 90 %, 95 %, 98 % or 99 % or more homology.

The phrase "agrochemicals having the capability to induce SAR" as used hereinabove and hereinbelow includes all kinds of chemical compounds which induce SAR, in particular those which stimulate the defense system endogenous to the plant (immunization) with or without direct action on the phytopathogenic microorganisms.

These agrochemicals protect the treated plants from attack by harmful microorganisms, for example phytopathogenic fungi, bacteria and viruses.

These agrochemicals include benzoic acid, salicylic acid, polyacrylic acid and derivatives thereof.

Another group of SAR inducing agrochemicals are benzo-1,2,3-thiadiazole derivatives as for example disclosed by US Patents US 4,931,581 and US 5,229,384.

The known SAR inducing compounds are preferably used as reference compounds for the screening of novel agrochemical compounds.

Other agrochemicals encompassed by the present invention, in particular individual members of a synthetic combinatorial library, can be determined easily by assaying the test chemical with the aid of the methods according to this invention.

The agrochemicals may be applied in pure form, in solution or suspension, as powders or dusts, or in other conventional formulations used agriculturally. Such formulations may include solid or liquid carriers, that is, materials with which said agrochemical is combined to facilitate application to the plant, tissue, cell or tissue culture, or to improve storage, handling or transport properties. Examples of suitable carriers include silicates, clays, resins, alcohols, ketones, aliphatic or aromatic hydrocarbons, and the like. If formulated as conventional wettable powder, aqueous suspension or emulsion concentrate, the agrochemical formulation may include one or more conventional surfactants, either ionic or non-ionic, such as wetting agents, emulsifying or dispersing agents.

The agrochemicals may also be applied in combination with another agent, for example with an adjuvant, a herbicide, a fungicide, an insecticide or a fertilizer.

As a liquid formulation the agrochemical may be applied as a spray to plant leaves, stems or branches or to seeds before planting or to soil or other growing medium.

The agrochemical is applied in an amount and over a period of time sufficient to induce SAR effects. If applied to whole plants, as a rule, the agrochemicals are applied in a concentration of 0.1 to 1000 mg active ingredient per liter of soil volume in a soil drench process. If working in a cell or tissue culture system, also lower concentrations, now based on the volume of the culture medium, may be used. As a guideline 6 to 72 hours, in particular 12 to 48 hours are sufficient to detect a response.

In principle every conventional method of assaying the expression of genes can be used to monitor the response of the plant on the action of the agrochemical, such as Western blot or Northern blot analysis. If the expression which has to be detected occurs tissue-specific, the analysis will be performed on said specific tissue, e.g. in the leaves of a plant in the case of the detection of the expression of a *LOX2:Hv1:1* type gene fragment.

If there exists a constitutive low level expression of the said gene fragment in the plant or plant part in the absence of salicylate, the expression induced by salicylate or a SAR inducing compound or composition will be determined as an increase in expression compared to said constitutive low level expression. This assaying variant is also encompassed by step (b) of the method according to the invention.

Preferably, the analysis of the expression of the said gene fragment is carried out using a Northern blot type analysis, in particular with detectably labelled cDNAs of the said gene fragment, e.g. barley *LOX2:Hv:1*, or portions thereof. A preferred label is digoxigenin. In the present context it may be convenient to use a commercially available Northern blot kit such as the DIG-High Prime labelling Kit of Boehringer Mannheim, Germany. It will be possible to use *LOX2:Hv:1* cDNA probes or probes comprising portions of *LOX2:Hv:1* cDNA for a great number of plants including various cereals due to the substantial homology of lipoxygenase genes the expression of which being inducible by salicylate, but not by naturally occurring plant pathogens, among these plants.

In another preferred embodiment of the present invention the analysis of the expression of the said gene fragment is carried out using antibodies to detect the gene product of the said gene fragment, e.g. the *LOX2:Hv:1* protein, e.g. by performing a Western blot type analysis.

The assaying step (b) for assaying the expression of the said gene fragment may be carried out in a way
- to produce a result which enables the mere determination whether or not an expression has occurred;
- to enable the determination which of two or more samples shows a higher degree of expression of the said gene fragment compared to the other(s) ("semi-quantitative determination");
- to provide quantitative results as to the level of expression occurred ("quantatitve determination").

If care is taken to perform the assaying step (b) with identical or substantially identical amounts of nucleotide or protein material prepared from the step (a) treated plants or plant parts, thus enabling a semi-quantitative determination and quantitative determination of the level of expression of the said gene fragment, the method according to the invention may also be used
- for determining the concentration of an agrochemical which is capable of inducing SAR in plants in a sample;
- for determining whether an agrochemical exhibits a stronger SAR inducing effect than a SAR inducing reference compound; and
- for determining whether and, optionally, to what degree a compound or composition is capable of changing the SAR inducing effect of an agrochemical.

The method for determining the concentration of an agrochemical which is capable of inducing SAR in plants in a sample, is characterized in that it comprises the following steps:
(i) performing the method according to the invention comprising steps (a) and (b) as outlined above wherein in step (a) the plant or plant part is contacted with an aliquot of the sample;
(ii) determining the concentration of the SAR inducing agrochemical in the sample by comparing the result from step (i) with the results of corresponding tests performed with known concentrations of said agrochemical.

The method for determining whether an agrochemical exhibits a stronger SAR inducing effect than a SAR inducing reference compound, such as salicylate, is characterized in that it comprises the following steps:
(i) performing the method according to the invention comprising steps (a) and (b) as outlined above wherein in step (a) the plant or plant part is contacted with a specific concentration of the agrochemical;
(ii) performing the method according to the invention comprising steps (a) and (b) as outlined above wherein in step (a) the plant or plant part is contacted with an amount of the SAR inducing reference compound resulting in an equimolar concentration compared to the concentration of the agrochemical in step (i);
(iii) determining the relative level of the SAR inducing effect of the agrochemical by comparing the results from steps (i) and (ii).
Since any step (b) will be carried out using substantially equal amounts of nucleotide or protein material prepared from step (a) treated plant or plant part material for the assay, step (iii) will produce a semi-quantitative result in order to enable the determination which one of the agrochemical and the SAR inducing reference compound induces a higher expression of the said salicylate-inducible lipoxygenase gene fragment and correspondingly will produce a stronger induction of SAR in a plant. Alternatively, the results may also be evaluated to enable a quantitative assessment of the relative degree of expression induced by the agrochemical compared to the SAR inducing reference compound. This evaluation may be performed for example by scanning of the analytical gels or films, photographs or print-outs thereof by means of a densitometer or by analyzing relevant excised sections of analytical gels used for separating assay mixtures comprising radioactively labelled detection compounds (e.g. nucleotide probes or antibodies) in a liquid scintillation counter.

The method for determining whether and, optionally, to what degree a compound or composition is capable of changing the SAR inducing effect of an agrochemical, is characterized in that it comprises the following steps:
(i) performing the method according to the invention comprising steps (a) and (b) as outlined above wherein in step (a) the plant or plant part is contacted with a specific concentration of the agrochemical;
(ii) performing the method according to the invention comprising steps (a) and (b) as outlined above wherein in step (a) the plant or plant part is contacted with the same specific concentration of the agrochemical as in step (i) in the presence of the compound or composition;
(iii) determining the capability of the compound or composition for changing the SAR inducing effect of the agrochemical by comparing the results from steps (i) and (ii).
Since also in this method any step (b) will be carried out using substantially equal amounts of nucleotide or protein material prepared from step (a) treated plant or plant part material for the assay, step (iii) will produce a semi-quantitative result in order to enable the determination whether the compound or composition leads to an enhancement, reduction or even inhibition of the expression of the said salicylate-inducible lipoxygenase gene fragment and correspondingly will produce a stronger or reduced induction of SAR in a plant or will even abolish the induction of SAR. Alternatively, the results may be evaluated to enable a quantitative assessment of the relative degree of expression induced by a combination of the agrochemical and the compound or composition compared to the agrochemical alone. This evaluation may be performed for example by scanning of the analytical gels or films, photographs or print-outs thereof by means of a densitometer or by analyzing relevant excised sections of analytical gels used for separating assay mixtures comprising radioactively labelled detection compounds (e.g. nucleotide probes or antibodies) in a liquid scintillation counter.

The method for determining whether and, optionally, to what degree a compound or composition is capable of changing the SAR inducing effect of an agrochemical may further be utilized in a method for screening for compounds or compositions capable of changing the SAR inducing effect of an agrochemical.

The invention further pertains to a kit for use in the methods according to the present invention. Said kits are characterized in that they comprise the following components:
(a) one or more plant or plant parts comprising a gene fragment coding for a lipoxygenase (LOX) the expression of which being inducible by salicylate, but not by naturally occurring plant pathogens; and
(b) means for detecting the expression of the said gene fragment.

If the kit comprises one or more plant part, such as plant cells, said plant parts may be provided immobilized on a solid support. Suitable supports may i.a. be made of glass, specific polysaccharides, like agarose, or specific plastic materials such as polyacrylamide, polystyrene, polyvinylalcohol, silicones. Methods and techniques for immobilizing cells and tissue on such types of supports are within the skills of the skilled scientist. In a specific embodiment, in particular for the screening processes according to the present invention, the kit may comprise plates, such as microtiter plates, comprising a multiplicity of reaction wells which contain the said plant parts, in particular plant cells, preferably immobilized on the bottom and/or the walls of the wells.

The means for detecting the expression of the said gene fragment may comprise one or more nucleotide probe(s) such as detectably labelled cDNAs or cDNA fragments of the said gene fragment. In a preferred embodiment of the present invention and for use in a number of different plants including many cereals, the nucleotide probe(s) may comprise the nucleotide sequence of *LOX2:Hv:1* cDNA or of portions thereof. The detectable label may be, e.g., a fluorescent, chemiluminescent or radioactive label, and is in particular a digoxigenin molecule. Alternatively, for use in Western Blot type analyses, the means for detecting the expression of the said gene fragment will comprise an antibody which specifically reckognizes and binds to the gene product of the said gene fragment. This antibody will carry a detectable label, e.g. a fluorescent or chemiluminescent dye, a radioactive label or an enzymatically active moiety or structure or will be detectable by use of a secondary antibody specific for said first antibody which may also be provided, separately from said first antibody, in the kit.

Furthermore, said means may comprise one or more buffers for the assay reaction(s) and/or reagents for determining and, in particular, visualization of the assay result (e.g. in the case of detection of the enzymatic activity attached to the antibody), which may be provided separately from each other and from the probes or antibodies or in combined form or in form of combinations of some of said components.

Optionally, the kit may comprise further components, in particular as separate components, such as one or more buffers for incubating the test compound or composition with a plant part, agents for breaking up the cellular structure of the plant or plant part for enabling the reactions for detection of the expression of the said gene fragment or instructions for use of the kit.

To illustrate the invention, specific examples are set forth below. These examples are merely illustrations and are not to be understood as limiting the scope and underlying principles of the invention in any way. Various modifications of the invention in addition to those shown and described herein will become apparent to those skilled in the art from the following examples and foregoing description. Such modifications are also intended to fall within the scope of the appended claims.

### EXAMPLES

### Materials and Methods

### Plants, pathogens, and inoculation

Barley (Hordeum vulgare L.) mlo-resistant cultivar Salome as well as the susceptible cultivars Pallas and Manchuria were grown as described for example by Hause et al., J. Plant Physiol. 150, 127-132 (1997). Inoculation with conidia of *Blumeria graminis f. sp. hordei* race A6 (Wiberg, Hereditas 77, 89-148 (1974)) were performed at a density of 10 conidia per mm² with the primary leaf of seven-days-old seedlings at the onset of the light period.

### Treatment of plants and determination of jasmonates and salicylate

Segments of seven-days-old primary leaves were freshly harvested and either floated on water, freshly prepared (±) JM, or Na-salicylate.

DCINA (2,6-dichloroisonicotinic acid), formulated as 25 % active ingredient with a wettable powder (WP) carrier (Métraux et al., Advances in molecular genetics of plant microbe interactions. Vol. 1, Kluwer Academic Publishers, Dordrecht, The Netherlands, pp. 432-439 (1991)), was applied to 7-days-old barley seedlings as a soil drench at a final concentration of 10 mg active ingredient/L soil volume. BTH (benzo[1,2,3]-thiadiazole-7-carbothioic acid S-methyl ester) was applied at a concentration of 1 mM by spraying a formulation with WP in distilled water. Controls were treated with WP.

Jasmonates were determined by an ELISA as described by Lehmann et al., Planta 197, 156-162 (1995). In this assay jasmonic acid, its methyl ester and its amino acid conjugates were detected as a sum down to an amount of 0.1 pmoles per g f.w. Salicylate was determined by HPLC analysis using a fluorescence detection method.

### RNA extraction and Northern blot analysis

Total RNA was extracted as described for example by Hause et al., Plant Cell Physiol. 37, 641-649 (1996). Northern blot analysis was performed with digoxigenin-Iabelled cDNAs (DIG-High Prime labelling Kit, Boehringer Mannheim) of barley *LOX2:Hv:1* (Vörös et al., *loc. cit*) and barley *PR1b* (Bryngelsson et al., Mol. Plant-Microbe Int. 7, 267-275 (1994)).

### Example 1

### Induction of Expression of LOX2:Hv:1 mRNA with salicylic acid (SA)

*LOX2:Hv:1* specific mRNA accumulated in the variety Salome at about 18 to 24 h after onset of JM treatment (Vörös et al., *loc cit*.). *LOX2:Hv:1* expression is induced upon treatment with SA. The dose-response curves for SA treatment (Fig. 1) reveal a slightly higher sensitivity of *LOX2:Hv:1* for JM. No differences in the kinetics of *LOX2:Hv:1* transcript accumulation was detected upon treatment with 45 µM JM and 45 µM SA.

### Example 2

### Induction of Expression of LOX2:Hv:1 mRNA with different agrochemicals

SA and derivatives thereof activated SAR in barley against the powdery mildew fungus. SA, DCINA and BTH are plant activators which share common structural features (Görlach et al., Plant Cell 8, 625-643 (1996)). Hence, a similar mode of action of all three compounds was proposed. Therefore, DCINA and BTH were tested on their ability to induce *LOX2:Hv:1*. Treatment of barley seedlings with these plant activators resulted in the accumulation of LOX transcript both in the powdery mildew susceptible (Fig. 2) and in the mlo-resistant cultivar. In all experiments, *LOX2:Hv:1* was stronger induced by BTH than by DCINA.

### Example 3

### Pathogen-related Induction of LOX2:Hv:1 mRNA

Treatments of barley plants with SA or JM, respectively, may cause accumulation of endogenous jasmonates or SA, respectively. This may explain a LOX-mRNA accumulation as a result of an endogenous rise of jasmonates upon treatment with SA or vice versa. Vörös et al. (*loc. cit*.) showed, however, that *LOX2:Hv:1* is not inducible by an endogenous rise of jasmonates. In order to prove whether LOX induction by exogenously applied JM is preceded by an elevation of the endogenous level of SA, the amount of SA was measured upon JM treatment in the variety Salome. An elevated level of endogenous SA was not detected (basis level = 0.5 µg per g f.w.). This indicates that the LOX induction occurred in response to the exogenously applied compound. Likewise, upon treatment by SA, the level of endogenous jasmonates remained unchanged (basis level = 0.15 nMol per g fw.).
In a further experiment, the pathogen-related induction of *LOX2:Hv:1* was investigated by recording its mRNA accumulation upon inoculation of resistant and susceptible barley with B. *graminis f. sp. hordei* (Fig. 3). No *LOX2:Hv:1* mRNA was detectable in the first 7 days after inoculation, whereas mRNA coding for the *PR1b* protein significantly accumulated in the resistant and susceptible barley cultivar. The same result was obtained when barley plants were inoculated with the non-host pathogen B. *graminis fsp. tritici* . In contrast to certain LOX-isoenzymes of other plants, the barley *LOX2:Hv:1* is not a pathogenesis-related gene. Accordingly, a *PR1b* mRNA accumulation in response to JM treatment was not found and no endogenous rise of jasmonates was detectable at least within the epidermal cell attacked by the powdery mildew fungus (Hause et al., 1997, *loc. cit*.). Plant activators such as SA, DCINA and BTH strongly activate PR genes in wheat. Moreover, plant activators such as BTH activate LOX, but not pathogen related genes like PR1b in barley. The finding that *LOX2:Hv:1* is responsive to plant activators but not to plant pathogens such as the powdery mildew fungus, provides an efficient and easy screening method for agrochemicals which are capable of inducing SAR in plants.

## Claims

1. A method of testing an agrochemical for its capability to induce SAR in plants said method comprising:
(a) contacting a plant or plant part comprising a gene fragment coding for a lipoxygenase (LOX) the expression of which being inducible by salicylate, but not by naturally occurring plant pathogens, with the agrochemical; and
(b) assaying the expression of said gene fragment, wherein the expression of said gene fragment indicates that the agrochemical has the capability to induce SAR.

2. The method according to claim 1 wherein a plant is used in which the salicylate-inducible expression of the said gene fragment is tissue-specific.

3. The method according to claim 2 wherein a plant is used in which the salicylate-inducible expression of the said gene fragment specifically occurs in the plant leaves.

4. The method according to any one of claims 1 to 3 wherein the said gene fragment the expression of which being inducible by salicylate, but not by naturally occurring plant pathogens, is a gene fragment comprising a nucleotide sequence corresponding to the nucleotide sequence of barley *LOX2:Hv:1* cDNA, a gene fragment comprising a nucleotide sequence having substantial homology with the nucleotide sequence of *LOX2:Hv:1* cDNA, a variant thereof or a gene fragment comprising one or more portions of the aforementioned gene fragments and variants.

5. The method according to claim 4 wherein the said gene fragment consists of 3073 basepairs.

6. The method according to any one of claims 1 to 5 wherein the said plant or plant part is selected from the group consisting of a plant protoplast, a plant cell, a plant tissue, a developing plantlet, an immature whole plant and a mature whole plant.

7. The method according to any one of claims 1 to 6 wherein the said plant or plant part is a cereal, and in particular a barley plant or plant material.

8. The method according to any one of claims 1 to 7 wherein the assaying of the expression of the said gene fragment is carried out using a Northern blot type analysis.

9. The method according to claim 8 wherein the Northern blot type analysis is carried out with one or more digoxigenin labelled cDNAs or cDNA fragments of barley *LOX2:Hv:1*.

10. The method according to any one of claims 1 to 7 wherein the assaying of the expression of the said gene fragment is carried out using a Western blot type analysis.

11. A method for screening for agrochemicals having the capability to induce SAR in plants wherein the method according to any one of claims 1 to 10 is used.

12. The method according to claim 11 wherein the agrochemicals to be screened are provided in form of a combinatorial library of chemical compounds.

13. A method for determining the presence of compounds capable of inducing SAR in plants in a sample, said method comprising: performing the method according to any one of claims 1 to 10 wherein in step (a) the plant or plant part is contacted with an aliquot of the sample and wherein the expression of the said gene fragment indicates the presence of one or more compounds capable of inducing SAR in plants in the sample.

14. A method for determining whether an agrochemical exhibits a stronger SAR inducing effect than a SAR inducing reference compound comprising the following steps:
(i) performing the method according to any one of claims 1 to 10 wherein in step (a) the plant or plant part is contacted with a specific concentration of the agrochemical;
(ii) performing the method according to any one of claims 1 to 10 wherein in step (a) the plant or plant part is contacted with an amount of the SAR inducing reference compound resulting in an equimolar concentration compared to the concentration of the agrochemical in step (i);
(iii) determining the relative level of the SAR inducing effect of the agrochemical by comparing the results from steps (i) and (ii).

15. Kit for use in the methods according any one of claims 1 to 14 which comprises the following components:
(a) one or more plant or plant parts comprising a gene fragment coding for a lipoxygenase (LOX) the expression of which being inducible by salicylate, but not by naturally occurring plant pathogens; and
(b) means for detecting the expression of the said gene fragment.

16. A process for providing chemical compounds having the capability of inducing SAR in plants which comprises the steps of
(a) generating a synthetic combinatorial library of chemical compounds, and
(b) screening the compounds of said library with a method as claimed in claim 11.

17. A new compound having the capability of inducing SAR obtainable by a process according to claim 16.

18. Use of a compound according to claim 17 as agrochemical compound, and in particular for the induction of SAR in plants.
